# EUROPEAN PATENT SPECIFICATION

(11) **EP 4 353 283 B1**
(45) Date of publication and mention of the grant of the patent: **29.04.2026**
(21) Application number: 23202498.4
(22) Date of filing: 09.10.2023
(51) Int. Cl.: A61M 5/38, A61M 39/24, A61M 5/165

(54) **FILTER COMPRISING AN OVERPRESSURE VALVE FOR INFUSION MEDICAL LINES**
FILTER MIT ÜBERDRUCKVENTIL FÜR MEDIZINISCHE INFUSIONSLEITUNG
FILTRE COMPRENANT UNE SOUPAPE DE SURPRESSION POUR LIGNE DE PERFUSION MÉDICALE

(30) Priority: 11.10.2022 IT 202200020931
(43) Date of publication of application: 17.04.2024
(73) Proprietor: Industrie Borla S.p.A., 10024 Moncalieri (Torino) (IT)
(72) Inventor: GUALA, Gianni, 10133 Torino (IT)
(74) Representative: Buzzi, Notaro & Antonielli d'Oulx S.p.A.

(56) References cited:
- EP-A2- 1 442 761
- WO-A1-2015/174861
- WO-A1-2019/215516
- US-A- 5 186 431

## Description

### Field of the invention

The present invention relates to a filter for infusion medical lines and more particularly it relates to an IV filter.

These filters comprise in a known manner a box-like body provided with an inlet connector for a liquid to be filtered and with an outlet connector for the filtered liquid. Inside the box-like body there is arranged a plate-like element formed on whose at least one of the opposite faces there are formed ribs defining internal channels extending parallel between the inlet and outlet connectors, and on the ribs there is arranged a filtering hydrophilic membrane which separates the channels from an interspace delimited by a corresponding half-shell. The interspace is placed in communication with the inlet connector and the channels are placed in communication with the outlet connector. Furthermore, the interspace is placed in communication with the external by means of vent openings with associated hydrophobic membranes for deaerating the liquid.

### State of the prior art

A filter of this type is disclosed, for example, in the document WO2019/215516A1 in the name of the same Applicant, according to which the plate-like element is formed on both faces with longitudinal grooves on which there are arranged two filtering hydrophilic membranes with associated interspaces.

When using filters thus made, there may occur a backflow condition in which the liquid, instead of flowing from the inlet connector towards the outlet connector, is supplied in the reverse direction, that is from the outlet connector towards the inlet connector, flowing from the longitudinal grooves of the plate-like element to the or to each interspace flowing through - in the reverse direction - the filtering hydrophilic membrane/s. In this condition, if the pressure of the liquid is high, there is the risk that the or each hydrophilic membrane, which is typically fixed - for example by gluing or other appropriate systems - to the plate-like element, could detach from the latter or even break, and - as a result - limit or even nullify the filtering action thereof when the flow of the liquid from the inlet connector towards the outlet connector is restored.

### Summary of the invention

The object of the present invention is to solve the aforementioned technical problem, that is to prevent the filtering membrane/s from detaching from the plate-like element when the flow of the liquid is reversed from the outlet connector towards the inlet connector, jeopardising the normal operation of the filter.

According to the invention, this object is attained due to the fact that in a filter for medical lines of the type defined in the preamble of claim 1, an overpressure valve is incorporated within the box-like body of the filter and is operatively associated with the outlet connector.

In this manner, should the backflow pressure of the liquid from the outlet connector towards the inlet connector exceed the maximum value corresponding to the calibration of the overpressure valve, the excess pressure is absorbed and vented towards the inlet connector without acting on the hydrophylic membrane/s.

The overpressure valve according to a preferred embodiment of the invention, comprises a chamber formed in the plate-like element and containing an obturator consisting of an elastic membrane which controls the communication between the outlet connector and the channels of the plate-like element. The chamber is connected - on one side of the obturator - to the aforementioned outlet connector through a by-pass conduit, and - on the other side of the obturator - to the inlet connector.

### Brief description of the drawings

The invention will now be described in detail with reference to the attached drawings, provided purely by way of non-limiting example, wherein:
- figure 1 is a partial plan view of a filter according to the invention,
- figure 2 is a longitudinal sectional view according to line II-II of figure 1,
- figure 3 is a cross-sectional view according to line III-III of figure 1,
- figure 4 shows - in larger scale - the detail shown in figure 2,
- figure 5 is an exploded perspective view of the filter according to the invention, and
- figure 6 shows a variant of figure 2.

### Detailed description of the invention

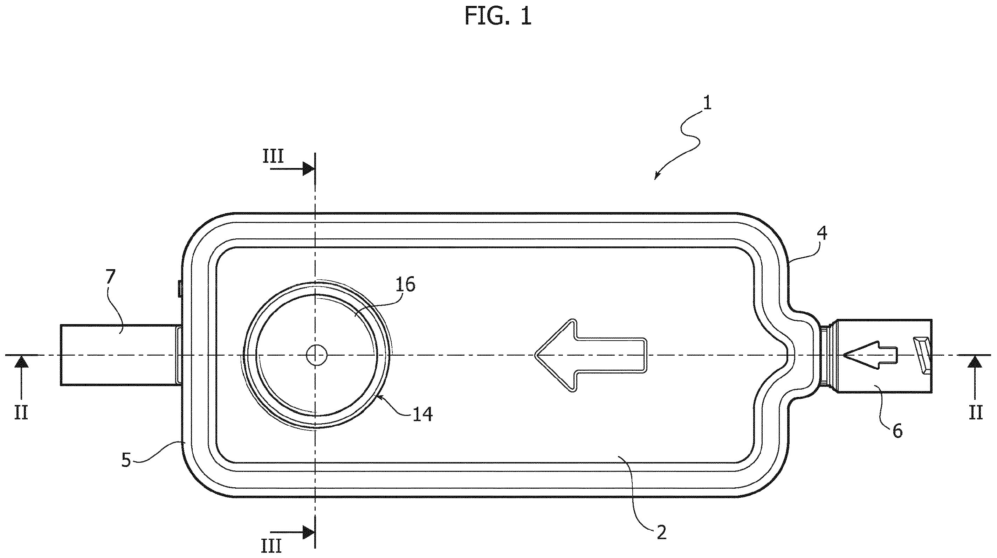

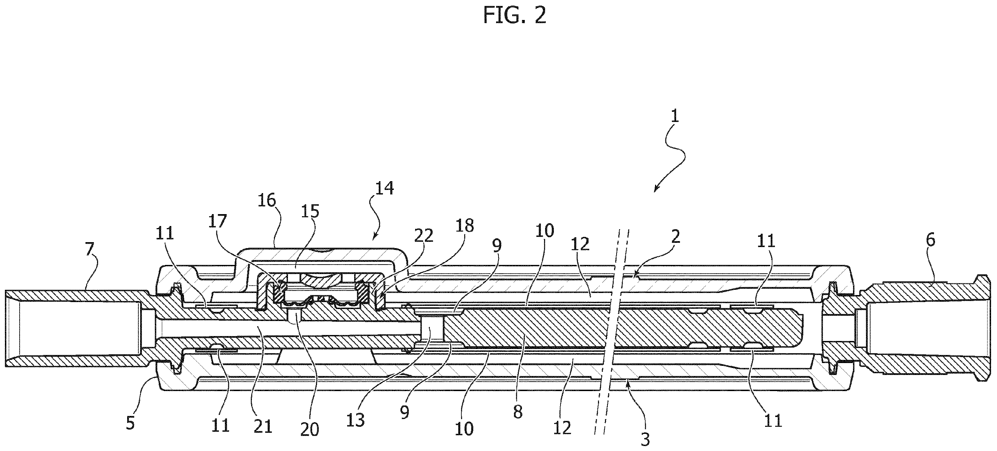

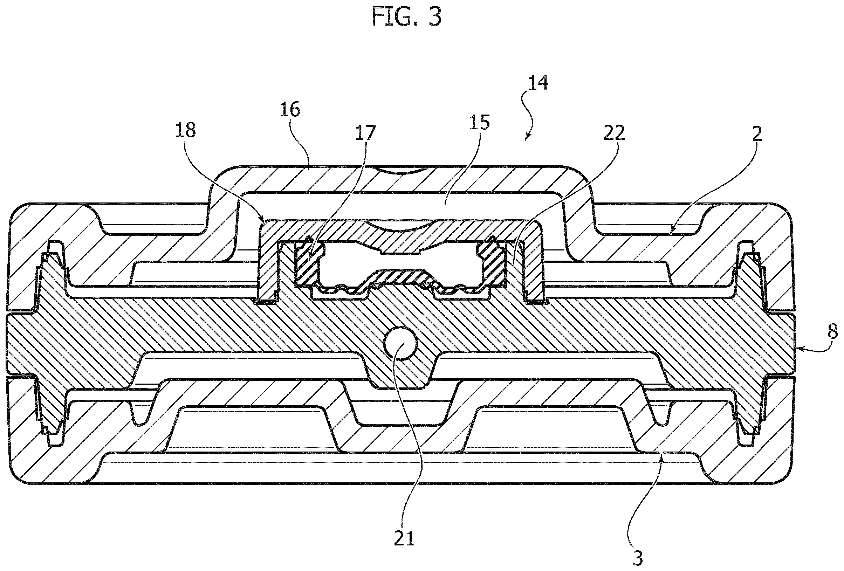

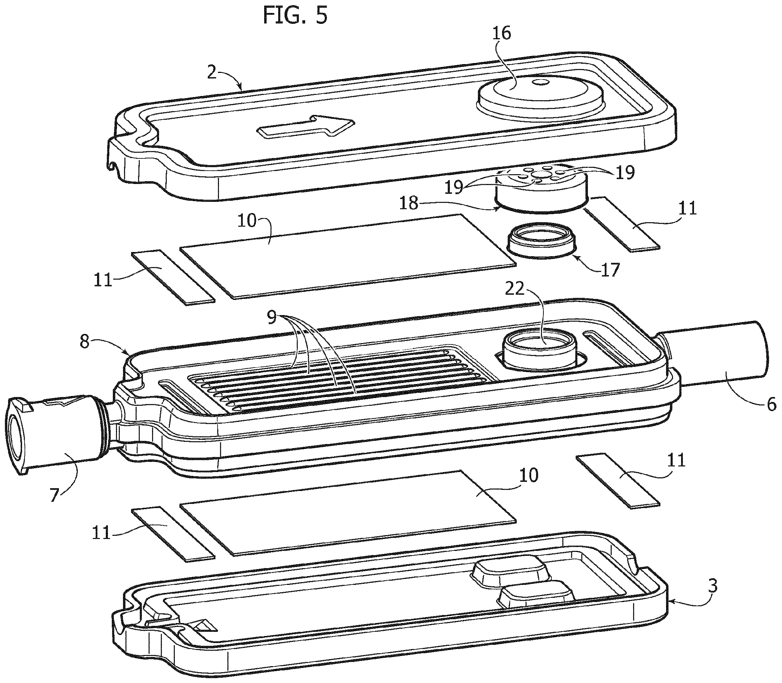

With reference to the figures 1-5 of the drawings - the IV filter according to the invention comprises a generally quadrangular-shaped box-like body formed - in the case of the shown example - by a pair of half-shells 2, 3 permanently joined to each other along the respective peripheral edges.

At the smaller side walls 4, 5 of the box-like body 1 there are respectively arranged a tubular inlet connector 6 and a tubular outlet connector 7 aligned to each other according to the longitudinal axis of the box-like body 1. The inlet connector 6 is designated, in use, to be connected to a duct for supplying a liquid to be filtered, and the outlet connector 7 is in turn designed to be connected to a duct for supplying the filtered liquid.

As observable hereinafter, when using the filter, there may occur a backflow condition in which the liquid flows in the reverse direction, that is from the outlet connector 7 towards the inlet connector 6, instead of flowing from the inlet connector 6 towards the outlet connector 7.

A plate-like element interposed between the two half-shells 2, 3 and on at least one of its opposite faces there are formed respective pluralities of ribs which define respective channels 9 which extend longitudinally between the connectors 6, 7, is indicated with reference numeral 8. In the case of the shown example, both faces of the sheet-like body 8 are formed with channels 9.

On each face of the plate-like element 8 there rests and there is fixed, for example by gluing or using an appropriate welding system, a respective hydrophilic membrane 10 on whose ends there is operatively associated a respective pair of hydrophobic membranes. It should be observed that the filter could be provided with a single filtering hydrophilic membrane 10, or even a number of filtering membranes greater than two.

Between the two hydrophylic membranes 10 and the half-shells 2, 3 there are defined respective longitudinal interspaces 12 which communicate - on one side - with the inlet connector 6 and - on the opposite side - with a cross-sectional passage 13, interposed between the hydrophylic membranes 10, in turn placed in communication with the outlet connector 7 through a longitudinal conduit 21.

During use, the infusion liquid is supplied to the filter by the inlet connector 6 (which can be configured for tube-tube or luer-tube or tube-luer or luer-luer connections of the medical line), it is split in the two interspaces 12 therefore touching and flowing through the filtering hydrophilic membranes 10 and then conveyed from the channels 9 towards the passage 13 and flow out from the filter through the conduit 21 and the outlet connector 7. The hydrophobic membranes 11 allow the air - possibly trapped in the liquid - to flow out from the filter through respective holes provided for on the end walls 4, 5 of the box-like body 1.

As mentioned above, when using the filter according to the invention, there may occur backflow situations that is the reverse flow of the liquid, flowing in from the outlet connector 7 and flowing out from the inlet connector 6. In situations of this kind, the pressure of the liquid flowing into the outlet connector 7 could be too high, and in particular so high to an extent of resulting in the risk of detachment of the filtering hydrophilic membranes 10 from the walls of the plate-like element 8, and even breakage. In this event, following the restoration of the flow of the liquid from the inlet connector 6 towards the outlet connector 7, the filter would be ineffective given that the correct filtration of the liquid would be jeopardised.

Therefore, the invention envisages providing the filter with an overpressure valve, or a by-pass valve, generally indicated with reference numeral 14.

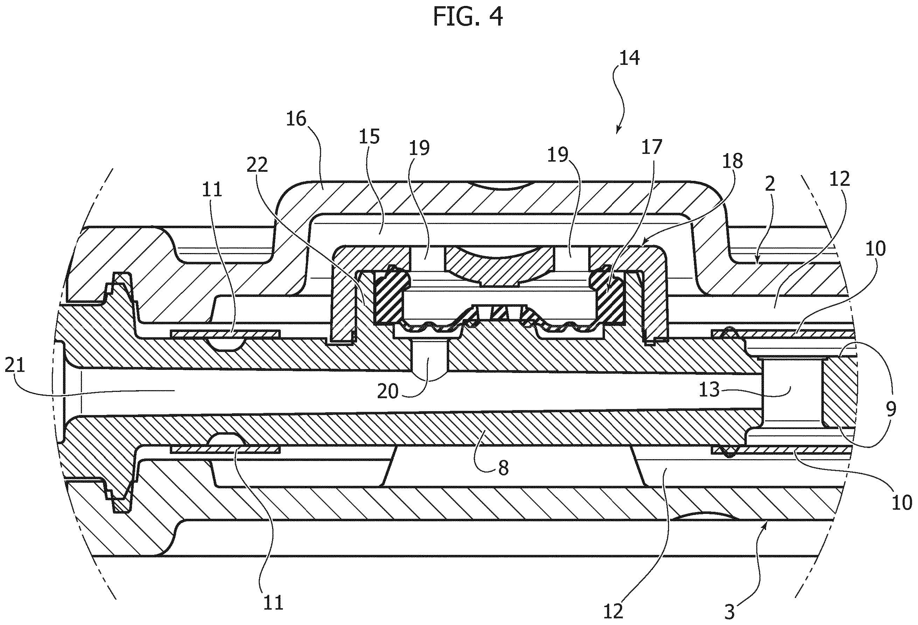

In greater detail, with reference to figure 4 the overpressure valve 14 is incorporated in the box-like body 1 of the filter and it comprises a chamber 15 formed between a substantially dome-like part 16 of the wall 2 and the corresponding surface of the plate-like element 8 proximal to the outlet connector 7. Inside the chamber 15 there is provided for an annular relief 22 in which there is arranged an elastic membrane 17 held in position by a drinking-glass-like element 18 formed with a crown of axial holes 19. The elastic membrane 17 controls the communication between the holes 19, in turn placed in communication with the inlet connector 6 through one and the other interspace 12, and a by-pass passage 20 placed in communication with the outlet connector 7 through the longitudinal conduit 21.

The overpressure valve 14 operates as follows.

When the filter operates in conditions of backflow from the outlet connector 7 towards the inlet connector 6, the liquid flows through the conduit 21 and the passage 13 flows into the channels 9 and, through the two hydrophylic membranes 10, it reaches the interspaces 12 and - from the latter - the inlet connector 6. Should the pressure of the liquid which flows through the conduit 21 be higher than a predetermined threshold value, corresponding to the calibration of the elastic membrane 17, the pressure acting in the passage 20 caused the opening elastic deformation of such membrane 17 and the ensuing flow of liquid from the by-pass passage 20 to the chamber 15, through the holes 19 of the retention element 17 and therefore, directly through the interspaces 12, towards the inlet connector 6. Therefore, the pressure drop of the liquid flowing in from the outlet connector 7 prevents the membrane 10 from being pushed towards the respective interspaces 12 to a point of detaching from the plate-like element 8 or even breaking.

When the pressure of the liquid flowing in from the outlet connector 7 returns below the threshold value, the elastic membrane 17 returns to the closing position, therefore the flow continues normally from the outlet connector 7 to the interspaces 12, flowing through the two hydrophylic membranes 10, and therefore to the inlet connector 6.

When the normal filtration flow of the liquid from the inlet connector 6 towards the outlet connector 7 is restored, the overpressure valve 14 normally remains inoperative.

Obviously, the construction details and the embodiments could widely vary with respect to what has been described and illustrated, without departing from the scope of protection of the present invention as defined in the claims that follow. Therefore, as mentioned above, the filter could be equipped with more than two filtering hydrophilic membranes 10, for example four, and even more than one overpressure valve 14.

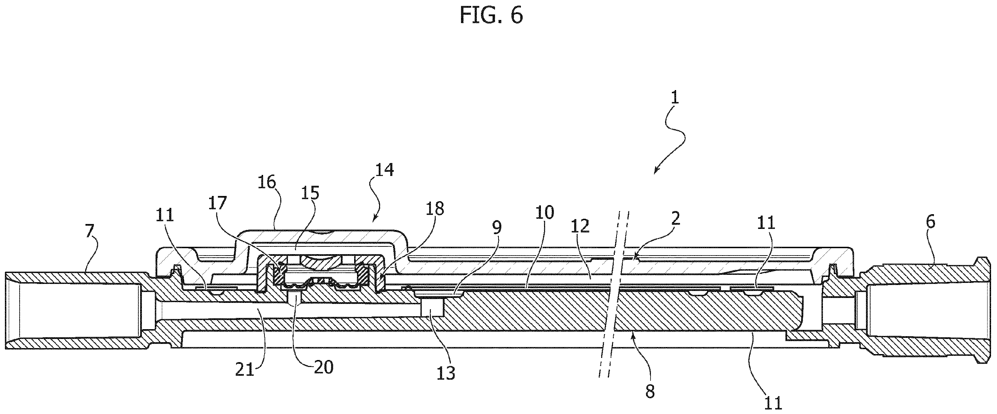

Should the variant shown in figure 6, wherein parts identical or similar to the ones already described are indicated with the same reference numerals, the filter provides for a single filtering hydrophilic membrane 10. In this case, the box-like body 1 comprises the half-shell 2 alone, and it is closed - at the lower part - by the plate-like element 8 whose longitudinal grooves 9 are formed on a single face. The inlet and outlet connectors 6, 7 are made as a single piece with the plate-like element 8, and the configuration and the operation of the overpressure valve 10 associated with the outlet connector 7 are similar to the description outlined above.

## Claims

1. Filter for medical infusion lines comprising a box-like body (1) having an inlet connector (6) for a liquid to be filtered and an outlet connector (7) for the filtered liquid, in the box-like body (1) there being provided for a plate-like element (8), on whose at least one of the opposite faces there are formed ribs, the ribs are formed defining internal channels (9) extending parallel between said connectors(6, 7), on said plate-like element (8) there being arranged at least one filtering hydrophilic membrane (10) which separates said channels (9) from at least one internal interspace (12), said interspace (12) being placed in communication with the inlet connector (6) and said channels (9) being placed in communication with the outlet connector (7), and said interspace (12) being further placed in communication with the external through hydrophobic membranes (11), **characterised in that** an overpressure valve (14) is incorporated within the box-like body (1) of the filter and is operatively associated to said outlet connector (7).

2. Filter according to claim 1, **characterised in that** said overpressure valve (14) comprises a chamber (15) formed in said plate-like element (8) and containing an obturator (17) which controls the communication between said outlet connector (7) and said inlet connector (6).

3. Filter according to claim 2, **characterised in that** said obturator (17) consists of an elastic membrane.

4. Filter according to claim 2 or 3, **characterised in that** said chamber (15) is connected - on one side - to said outlet connector (7) through a by-pass passage (20) and - on the other side - to said interspace (12).

## Patentansprüche

1. Filter für medizinische Infusionsleitungen, umfassend einen kastenartigen Körper (1) mit einem Einlassanschluss (6) für eine zu filternde Flüssigkeit und einem Auslassanschluss (7) für die gefilterte Flüssigkeit, wobei in dem kastenartigen Körper (1) ein plattenartiges Element (8) vorgesehen ist, auf dessen mindestens einer der gegenüberliegenden Seiten Rippen ausgebildet sind, wobei die Rippen ausgebildet sind, um innere Kanäle (9) zu definieren, die sich parallel zwischen den Anschlüssen (6, 7) erstrecken, wobei auf dem plattenartigen Element (8) mindestens eine filternde hydrophile Membran (10) angeordnet ist, welche die Kanäle (9) von mindestens einem inneren Zwischenraum (12) trennt, wobei der Zwischenraum (12) in Verbindung mit dem Einlassanschluss (6) platziert ist und die Kanäle (9) in Verbindung mit dem Auslassanschluss (7) platziert sind, und wobei der Zwischenraum (12) ferner in Verbindung mit dem Äußeren durch hydrophobe Membranen (11) platziert ist, **dadurch gekennzeichnet, dass** ein Überdruckventil (14) in den kastenartigen Körper (1) des Filters integriert ist und betriebsfähig mit dem Auslassanschluss (7) verbunden ist.

2. Filter nach Anspruch 1, **dadurch gekennzeichnet, dass** das Überdruckventil (14) eine Kammer (15) umfasst, die in dem plattenartigen Element (8) ausgebildet ist und einen Obturator (17) enthält, der die Verbindung zwischen dem Auslassanschluss (7) und dem Einlassanschluss (6) steuert.

3. Filter nach Anspruch 2, **dadurch gekennzeichnet, dass** der Obturator (17) aus einer elastischen Membran besteht.

4. Filter nach Anspruch 2 oder 3, **dadurch gekennzeichnet, dass** die Kammer (15) - auf einer Seite - mit dem Auslassanschluss (7) durch einen Umgehungsdurchgang (20) und - auf der anderen Seite - mit dem Zwischenraum (12) verbunden ist.

## Revendications

1. Filtre pour lignes de perfusion médicale comprenant un corps en forme de boîte (1) ayant un connecteur d'entrée (6) pour un liquide à filtrer et un connecteur de sortie (7) pour le liquide filtré, dans le corps en forme de boîte (1) est prévu un élément en forme de plaque (8) sur au moins l'une des faces opposées duquel sont formées des nervures, les nervures sont formées en définissant des canaux internes (9) s'étendant parallèlement entre lesdits connecteurs (6, 7), sur ledit élément en forme de plaque (8) étant disposée au moins une membrane hydrophile de filtration (10) qui sépare lesdits canaux (9) d'au moins un espace intermédiaire interne (12), ledit espace intermédiaire (12) étant placé en communication avec le connecteur d'entrée (6) et lesdits canaux (9) étant placés en communication avec le connecteur de sortie (7), et ledit espace intermédiaire (12) étant en outre placé en communication avec l'extérieur par l'intermédiaire de membranes hydrophobes (11), **caractérisé en ce qu'**une soupape de surpression (14) est incorporée dans le corps en forme de boîte (1) du filtre et est fonctionnellement associée audit connecteur de sortie (7).

2. Filtre selon la revendication 1, **caractérisé en ce que** ladite soupape de surpression (14) comprend une chambre (15) formée dans ledit élément en forme de plaque (8) et contenant un obturateur (17) qui contrôle la communication entre ledit connecteur de sortie (7) et ledit connecteur d'entrée (6).

3. Filtre selon la revendication 2, **caractérisé en ce que** ledit obturateur (17) consiste en une membrane élastique.

4. Filtre selon la revendication 2 ou 3, **caractérisé en ce que** ladite chambre (15) est connectée - sur un côté - audit connecteur de sortie (7) par un passage de dérivation (20) et - sur l'autre côté - audit espace intermédiaire (12).
